# EUROPEAN PATENT APPLICATION

(11) **EP 3 088 008 A1**
(43) Date of publication of application: **02.11.2016**
(21) Application number: 14873568.1
(22) Date of filing: 25.12.2014
(51) Int. Cl.: A61L 15/16, A61K 9/10, A61K 9/12, A61K 9/70, A61K 31/716, A61K 31/717, A61K 31/718, A61K 31/721, A61K 31/722, A61K 31/732, A61K 31/733, A61K 47/18, A61K 47/32, A61K 47/36, A61K 47/38, A61K 47/40, A61K 47/42, A61P 7/04, A61P 17/02

(54) **AQUEOUS DISPERSION FOR SOLIDIFYING SERUM AND BLOOD**

(30) Priority: 25.12.2013 JP 2013266931
(71) Applicant: Nissan Chemical Industries, Ltd., Chiyoda-ku Tokyo 101-0054 (JP)
(72) Inventor: IWAMA, Takehisa, Funabashi-shi Chiba 274-8507 (JP); HAYASHI, Hisato, Funabashi-shi Chiba 274-8507 (JP)
(74) Representative: Hoffmann Eitle
(86) International application number: PCT/JP2014/084397
(87) International publication number: WO 2015/099083

(57) **Abstract**

There is provided a novel hemostatic material (aqueous dispersion) that can be applied by a simple method of application in the form of a liquid or a spray mist to a bleeding wound surface, exhibits an excellent solidifying action on all of plasma, serum, and blood, and achieves a hemostatic effect quickly and easily. An aqueous dispersion comprising insoluble polysaccharides having an average fiber diameter (D) of 0.001 to 100 µm and a ratio (L/D) of an average fiber length (L) to an average fiber diameter (D) of 5 to 500, characterized in that the aqueous dispersion causes plasma, serum, or blood to solidify under contact with plasma, serum, or blood.

## Description

### TECHNICAL FIELD

The present invention relates to an aqueous dispersion containing insoluble polysaccharides. In particular, the present invention relates to an aqueous dispersion that contains insoluble fibrous polysaccharides having a small fiber diameter and a comparatively small aspect ratio, causes plasma, serum, or blood to solidify under contact with plasma, serum, or blood, and is useful as a hemostatic material.

### BACKGROUND ART

As a material having an effect of causing coagulation under contact with blood or the like, that is, a hemostatic effect, a thrombin powder, collagen, a cellulose oxide powder, collagen fiber and powder, a collagen sponge, calcium alginate, or the like, has been known. Such a hemostatic material is used as a hemostatic in various forms of a hemostatic powder, a hemostatic sponge (collagen sponge, etc.), and a hemostatic gauze, and after hemostasis, the hemostatic material is usually removed.

When cellulose that is one of insoluble polysaccharides is subjected to chemical modification such as alkylation and oxidation, the resultant product is used as a water-soluble polymer in a wide field including foods, cosmetics, and pharmaceuticals. For example, carboxymethyl cellulose sodium (CMC) that is a typical water-soluble cellulose derivative is processed into the form of non-woven fabric. This CMC has been proposed as a hemostatic material in which bleeding is delayed and prevented by absorbing water, blood, or body fluid (Patent Documents 1 and 2, etc.).

Since an amino group in chitosan (β-1,4-N-acetyl-D-glucosamine) that is an insoluble polysaccharide acts on a blood coagulation system to exhibit a hemostatic effect, chitosan is used for clinical applications including a hemostatic in which chitosan is processed into the form of non-woven fabric. It has been pointed out that chitin (β-1,4-N-acetyl-D-glucosamine) that is an acetylated form of chitosan produce inflammation in the body. However, it has been reported that sponge-shaped amorphous chitin causes formation of platelet thrombus to exhibit a hemostatic action in a short time (Non-Patent Document 1).

Further, a hydrogel of a high molecular weight peptide (self-assembling peptide) containing laminin, collagen, and nidogen that are natural proteins as motifs (Patent Document 3) has attracted attention as a topical hemostatic.

As an external skin preparation effective in injury with bloodshed or exudation of body fluid, or the like, an external preparation composition containing a hydrophilic colloidal water-soluble polymer such as starch and gelatin and a liquid hydrocarbon compound has been proposed (Patent Document 4).

### Prior Art Documents

### Patent Documents

Patent Document 1: International Publication WO 98/46818
Patent Document 2: Japanese Translation of PCT International Application Publication No. 2003-510475 (JP 2003-510475 A)
Patent Document 3: Japanese Patent No. 5057781 (JP 5057781 B2)
Patent Document 4: Japanese Patent Application Publication No. 2001-97848 (JP 2001-97848 A)

### Non-Patent Documents

Non-Patent Document 1: JOURNAL OF THE KYORIN MEDICAL SOCIETY vol. 44, No. 1, 3-11, 2013

### SUMMARY OF THE INVENTION

### Problem to be Solved by the Invention

However, various hemostatics that have been proposed do not have a sufficiently satisfied hemostatic effect. Therefore, the hemostatics may require concomitant use with another topical hemostatic, or be easily separated from a hemostatic area to increase the risk of infection at the area. The hemostatics may express the hemostatic effect on any one of plasma, serum, and blood, but do not sufficiently express the hemostatic effect on some subjects. When some of the hemostatics absorb such body fluid, the strength of the hemostatics is decreased to lose the product shape. In addition, when a raw material derived from an animal such as human-derived fibrin and cattle-derived collagen is used as a hemostatic material, a xenobiotic reaction, infection with hepatitis C virus or the like that is generated from the animal-derived material, or an allergic reaction may be caused.

Since the aforementioned self-assembling peptide has an acidic pH of 3, cells and tissues may be injured during attachment of the peptide. Since the cost of raw materials for peptide synthesis is expensive, a problem arises in terms of general purpose. Further, there have been many problems in which information about a side effect of immune reaction against a foreign substance and the like have not been obtained.

Therefore, a novel hemostatic material that can appropriately suppress bleeding, and is free from the risk of infection, inexpensive, and safe is desired.

In recent years, insoluble cellulose itself that is converted into an aqueous dispersion is investigated for adjustment of strength of a sheet or a film or application to cosmetics, but the application of the aqueous dispersion to clinical application has not been tried.

It is an object of the present invention to provide a novel hemostatic material (aqueous dispersion) that can be applied by a simple method of application in the form of a liquid or a spray mist to a bleeding wound surface, exhibits an excellent solidifying action on all of plasma, serum, and blood, and achieves a hemostatic effect quickly and easily.

### Means for Solving the Problems

The inventor of the present invention has intensively studied the problems. As a result, the inventor has found that an aqueous dispersion in which an insoluble natural polysaccharide such as cellulose is dispersed in water quickly causes blood to solidify under contact with blood, that is, has a hemostatic effect. The present invention has been accomplished.

As a first aspect, the present invention relates to an aqueous dispersion comprising insoluble polysaccharides having an average fiber diameter (D) of 0.001 to 100 µm and a ratio (L/D) of an average fiber length (L) to an average fiber diameter (D) of 5 to 500, characterized in that the aqueous dispersion causes plasma, serum, or blood to solidify under contact with plasma, serum, or blood.

A second aspect relates to the aqueous dispersion according to the first aspect, wherein the insoluble polysaccharides are selected from the group consisting of cellulose fibers and chitin fibers.

A third aspect relates to the aqueous dispersion according to the first or second aspect, wherein the insoluble polysaccharides are contained in a concentration of 0.001% by mass to 10% by mass.

A fourth aspect relates to the aqueous dispersion according to any one of the first to third aspects, further comprising a polymer.

A fifth aspect relates to the aqueous dispersion according to the fourth aspect, wherein the polymer is selected from the group consisting of collagen, alginic acid, polyvinyl alcohol, carboxymethyl cellulose, methyl cellulose, and hyaluronic acid.

A sixth aspect relates to the aqueous dispersion according to any one of the first to fifth aspects, further comprising a functional component.

A seventh aspect relates to the aqueous dispersion according to the sixth aspect, wherein the functional component is selected from the group consisting of ascorbic acid, aminocaproic acid, tranexamic acid, and thrombin.

An eighth aspect relates to the aqueous dispersion according to any one of the first to seventh aspects, wherein the aqueous dispersion is capable of spraying, and the spraying causes plasma, serum, or blood to solidify.

A ninth aspect relates to use of the aqueous dispersion according to any one of the first to eighth aspects in hemostasis application.

A tenth aspect relates to use of the aqueous dispersion according to any one of the first to eighth aspects in wound healing application.

An eleventh aspect relates to use of the aqueous dispersion according to any one of the first to eighth aspects in adhesion preventing application.

A twelfth aspect relates to a film or a sheet obtained from the aqueous dispersion according to any one of the first to eighth aspects.

A thirteenth aspect relates to an external composition comprising the aqueous dispersion according to any one of the first to eighth aspects.

A fourteenth aspect relates to a hemostatic composition comprising the aqueous dispersion according to any one of the first to eighth aspects.

A fifteenth aspect relates to a wound healing composition comprising the aqueous dispersion according to any one of the first to eighth aspects.

A sixteenth aspect relates to an adhesion preventing composition comprising the aqueous dispersion according to any one of the first to eighth aspects.

### Effects of the Invention

The present invention can provide an aqueous dispersion that quickly causes plasma, serum, and blood to solidify under contact with plasma, serum, and blood and is useful as a hemostatic material.

Since insoluble polysaccharides used for a material for the aqueous dispersion of the present invention are inexpensive, the product cost can be reduced. Since any raw material derived from an animal is not used for the aqueous dispersion, the aqueous dispersion has no fear of causing infection with C virus or the like.

An insoluble polysaccharide can be converted into an aqueous dispersion, for example, by micronization through a wet milling method without chemical modification. Therefore, the aqueous dispersion can prevent a decrease in safety due to residual of various drugs accompanied by chemical modification.

In addition, the aqueous dispersion of the present invention can be subjected to a sterilization treatment by an autoclave or the like, is not corroded, and can achieve the hemostatic effect by a simple method in the form of a liquid (dispersion) or a spray mist without being processed into the form of a powder, a thread, a non-woven fabric, or a woven fabric.

Blood in the blood vessel is usually in a state in which balance between coagulation and fibrinolysis is kept. When the balance is lost by various causes such as abnormality of the vessel wall, abnormality of platelet, abnormality of the coagulation system, and enhancement of the fibrinolysis system, bleeding tends to occur. This tendency is "bleeding tendency."

The aqueous dispersion containing insoluble polysaccharides of the present invention causes plasma, serum, or blood to physically solidify under contact with plasma, serum, or blood to exhibit a hemostatic action. Therefore, the aqueous dispersion can exhibit the hemostatic action regardless of bleeding causes such as abnormality of the coagulation system and enhancement of the fibrinolysis system. Accordingly, the aqueous dispersion can exhibit a hemostatic effect even on bleeding not only during occurrence of an injury but also during surgery.

### BRIEF DESCRIPTION OF THE DRAWINGS

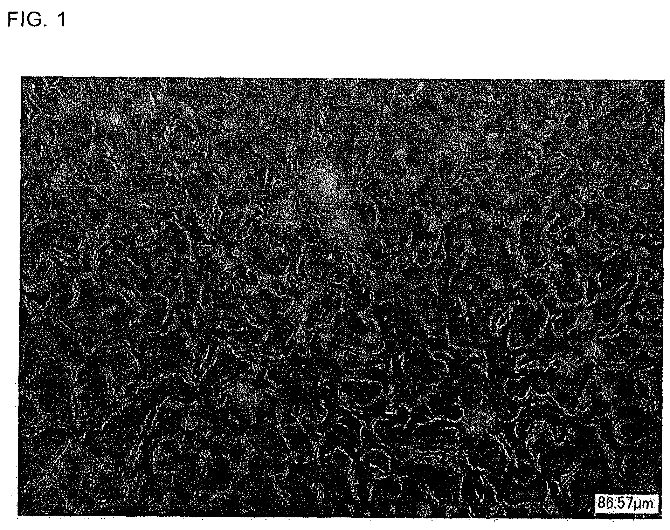

[FIG. 1] FIG. 1 is a view showing an optical micrograph of rabbit blood after addition of an aqueous dispersion of 1% pulp-derived cellulose in Example 4.

### MODES FOR CARRYING OUT THE INVENTION

### <Insoluble Polysaccharides>

Examples of insoluble polysaccharides used for the aqueous dispersion of the present invention include cellulose, chitin, cyclodextrin, and dietary fibers. Further, lignin, hemicellulose, inulin, protopectin, glucan, glucomannan, chemically modified cellulose oxide, or the like, may be used. In particular, it is preferable that the insoluble polysaccharides be selected from insoluble cellulose fibers and chitin fibers.

### [Cellulose Raw Material]

As a raw material for cellulose fibers used in the aqueous dispersion of the present invention, for example, cellulose derived from plants such as wood, bamboo, hemp, jute, kenaf, cotton, farm products, and scraps of food, or cellulose produced by microorganisms or animals, such as bacterial cellulose, Cladophoraceae (Cladophora), Glaucophytes (Glaucocystis), Valonia, and Hoya cellulose can be used.

Cellulose derived from plants forms a higher order structure having fibril, lamella, and desmacyte in a stepwise manner with bundles of very thin fibers called microfibrils. On the other hands, bacteria cellulose forms a fine network structure with the size of microfibrils of cellulose secreted from mycetocytes remained the same.

A crystalline substance of naturally-occurring cellulose such as the aforementioned cellulose derived from plants, or produced by microorganisms or animals is composed of a cellulose I type crystal, and the crystallinity largely varies depending on cellulose sources. The plant-derived cellulose forms a higher order structure containing impurities such as hemicellulose and lignin. Therefore, purified pulp obtained from the plant-derived cellulose as a raw material has a crystallinity of about 50%. On the other hands, purified pulp obtained from Cladophora, bacterial cellulose, Glaucocystis, or Hoya has a crystallinity as high as 80% or more.

In the present invention, a high-purity cellulose raw material such as cotton and bacterial cellulose may be used as it is. It is preferable that the plant-derived cellulose other than the high-purity cellulose be used after isolation or purification.

It is preferable that the raw material for cellulose fibers used in the aqueous dispersion of the present invention be cotton, bacterial cellulose, kraft pulp, or microcrystalline cellulose.

### [Chitin Raw Material]

As a chitin raw material, a commercially available product such as a food additive, a pharmaceutical additive, a quasi drug, a cosmetic raw material, and a medical equipment raw material that are contained in shrimps, crabs, squids, insects, shells, or mushrooms can be suitably used.

### [Methods for Pulverizing Cellulose and Chitin]

In the present invention, cellulose fibers obtained by pulverizing the cellulose raw material or chitin fibers obtained by pulverizing the chitin raw material are used. Methods for pulverizing the cellulose raw material and the chitin raw material are not particularly limited. In order to micronize the cellulose raw material and the chitin raw material into a fiber diameter and a fiber length (to be discussed later) that satisfy the object of the present invention, a method of achieving a high shear force such as a media agitating mill including a high-pressure homogenizer, a grinder (stone mill), and a bead mill is preferred.

Among them, it is preferable that a high-pressure homogenizer be used for micronization. For example, micronization (pulverization) through a wet pulverizing method described in Japanese Patent Application Publication No. 2005-270891 (JP 2005-270891 A) is desired. Specifically, a dispersion in which the cellulose raw material or the chitin raw material is dispersed is sprayed at high pressure from a pair of nozzles and made to collide to pulverize the cellulose raw material. For example, the micronization can be carried out using Star Burst system (high-pressure pulverizing device manufactured by Sugino Machine Limited).

When the cellulose raw material or the chitin raw material is micronized (pulverized) using the high-pressure homogenizer, the degrees of micronization and homogenization depend on a pressure that is applied so as to transfer the material to an ultra-high pressure chamber in the high-pressure homogenizer, the number of passages of the material through the ultra-high pressure chamber (the number of processes), and the concentration of cellulose or chitin in the aqueous dispersion.

The transfer pressure (process pressure) is usually 50 to 250 MPa, and preferably 150 to 245 MPa. When the transfer pressure is less than 50 MPa, micronization of cellulose or chitin is not sufficient, and an expected effect to the micronization is not obtained.

The concentration of cellulose or chitin in the aqueous dispersion during a micronization process is 0.1% by mass to 30% by mass, and preferably 1% by mass to 10% by mass. When the concentration of cellulose or chitin in the aqueous dispersion is less than 0.1% by mass, the productivity is significantly low, and when the concentration is more than 30% by mass, the pulverization efficient is low. Therefore, desired cellulose fibers and chitin fibers are not obtained.

The number of micronization (pulverization) processes is not particularly limited, and depends on the cellulose concentration/the chitin concentration in the aqueous dispersion. When the cellulose concentration/the chitin concentration is 0.1 by mass to 1% by mass, about 10 to 100 processes achieve sufficient micronization. However, when the concentration is 1 by mass to 10% by mass, about 10 to 1,000 processes are required. When the concentration is higher than 30% by mass, several thousands or more processes are required, and the viscosity increases to a viscosity at which the handling is difficult. Therefore, this is not realistic from the industrial viewpoint.

The average fiber diameter (D) of the cellulose fibers or the chitin fiber used in the present invention is 0.001 µm to 100 µm, preferably 0.001 µm to 0.05 µm, and more preferably 0.01 µm to 0.05 µm. When the average fiber diameter is less than 0.001 µm, the cellulose fibers or the chitin fibers are too thin, and the addition effect is not obtained. That is, the aqueous dispersion containing the cellulose fibers or the chitin fibers causes plasma, serum, or blood not to sufficiently solidify under contact with plasma, serum, or blood (for example, syneresis). When the average fiber diameter is more than 100 µm, the cellulose fibers or the chitin fibers are not sufficiently micronized, precipitation and aggregation are likely to occur in the aqueous dispersion, and spray properties may be deteriorated.

The aspect ratio (L/D) of the cellulose fibers or the chitin fibers used in the present invention is determined from a ratio of average fiber length (L)/average fiber diameter (D), and is 5 to 500, preferably 10 to 500, and more preferably 20 to 110. When the aspect ratio is less than 5, the dispersibility of the fibers in the liquid is not sufficient, and coating properties during drying are not sufficiently obtained. An aspect ratio of more than 500 means that the fiber length is extremely large. This leads to a decrease in the transparency, and further leads to degradation in the feeling of transparency and the feeling of use during application to the skin, and occurrence of clumping.

In the present invention, the average fiber diameter (D) of cellulose or chitin was determined as follows. A collodion-support film available from Okenshoji Co., Ltd., was first subjected to a hydrophilic treatment for 3 minutes using an ion cleaner (JIC-410) manufactured by JEOL Ltd., and several droplets of a cellulose dispersion or a chitin dispersion (diluted with ultrapure water) produced in Production Examples were added to the film, and dried at room temperature. This film was observed at an accelerating voltage of 200 kV with a transmission electron microscope (TEM, H-8000) (10,000-fold) manufactured by Hitachi, Lid. From a resulting image, the fiber diameter of each of 200 to 250 cellulose fibers or chitin fibers was measured, and the average thereof was determined as the average fiber diameter (D).

For the average fiber length (L), the cellulose dispersion or the chitin dispersion produced in Production Examples was diluted to 400 times by volume with dimethyl sulfoxide (DMSO), to disperse cellulose or chitin. The resulting dispersion was casted on a silicon wafer of which the surface is subjected to a hydrophilic treatment using concentrated sulfuric acid in advance, and dried at 110°C for 1 hour, to prepare a sample. The fiber length of each of cellulose fibers or chitin fibers (sample number: 150 to 250) was measured from an image of the resulting sample with a scanning electron microscope (SEM, JSM-7400) (10,000-fold) manufactured by JEOL Ltd., and the average thereof was determined as the average fiber length (L). The average fiber diameter and the average fiber length of chitosan fibers used in Comparative Examples were determined by the same methods.

It is preferable that the insoluble polysaccharides be added in an amount of 0.001% by mass to 10% by mass, and preferably 0.01% by mass to 10% by mass with respect to the total amount of the aqueous dispersion.

One of the insoluble polysaccharides may be used alone or two or more thereof may be used in combination.

### <Polymer>

To the aqueous dispersion of the present invention, a polymer may be further added as long as the effect is not impaired. Examples thereof include water-soluble polymers.

Examples of naturally occurring water-soluble polymers include plant-based polymers (e.g., gum arabic, gum tragacanth, galactan, locust bean gum, guar gum, tamarind gum, carob gum, karaya gum, carrageenan, pectin, agar, quince seed, algae colloid (brown algae extract), starch (rice starch, corn starch, potato starch, and wheat starch), and glycyrrhizinic acid); microorganism-based polymers (e.g., xanthan gum, dextran, succinoglucan, and pullulan); animal-based polymers (e.g., collagen, casein, albumin, and gelatin); and hyaluronic acid.

Examples of semisynthesis water-soluble polymers include starch-based polymers (e.g., carboxymethyl starch, and methylhydroxypropyl starch); and cellulose-based polymer (methyl cellulose, ethyl cellulose, methylhydroxypropyl cellulose, hydroxyethyl cellulose, cellulose sodium sulfate, hydroxypropyl cellulose, carboxymethyl cellulose, carboxymethyl cellulose sodium, crystalline cellulose, and a cellulose powder); alginic acid-based polymers (e.g., alginic acid, sodium alginate, and propylene glycol alginate ester).

Examples of synthesis water-soluble polymers include vinyl-based polymers (e.g., polyvinyl alcohol, polyvinyl methyl ether, polyvinyl pyrrolidone, and carboxyvinyl polymer); polyoxyethylene-based polymers (e.g., polyethylene glycol 20,000, 40,000, and 60,000); acrylic polymers (e.g., sodium polyacrylate, polyethyl acrylate, and polyacrylamide); polyethyleneimines; and cationized polymers.

Among them, a polymer selected from the group consisting of collagen, alginic acid, polyvinyl alcohol, carboxymethyl cellulose (CMC), methyl cellulose, and hyaluronic acid is preferred.

For example, when polyvinyl alcohol used as a material for a wound dressing, or CMC or hyaluronic acid used as a material for an adhesion preventing agent is added, the resultant mixture can be used as a wound dressing material or an adhesion preventing agent.

When the polymer is added to the aqueous dispersion of the present invention, the amount of the polymer added is preferably 0.01% by mass to 10% by mass, and more preferably 0.1% by mass to 5% by mass with respect to the total amount of the aqueous dispersion of the present invention.

### <Functional Component>

To the aqueous dispersion of the present invention, a functional component described below may be further added as long as the effect is not impaired.

Examples of the functional component include acrinol, ascorbic acid, isopropanol, benzalkonium chloride, benzethonium chloride, diluted iodine tincture, chlorhexidine gluconate, ethanol, phenoxyethanol, povidone-iodine, mercurochrome, isopropyl methylphenol, ethyl aminobenzoate, methylparaben, glycerol, propylene glycol, butylene glycol, octanediol, carbazochrome sulfonic acid, diclofenac, glycyrrhetinic acid, indomethacin, prednisolone valerate acetate, ketoprofen, bufexamac, hydrocortisone, thymol, piroxicam, felbinac, vanillylamide nonanoate, glycol salicylate, salicylic acid, allantoin, dipotassium glycyrrhizinate, tocopherol acetate, lidocaine, sulfur, cetylpyridinium chloride, chlorpheniramine maleate, dibucaine hydrochloride, naphazoline hydrochloride, iodine, diphenhydramine, diphenhydramine hydrochloride, alum, menthol, camphor, pyridoxine hydrochloride, urea, vitamin A oil, benzyl nicotinate, crotamiton, capsicum tincture, mentha oil, undecylenic acid, zinc undecylenate, exalamide, clotrimazole, miconazole nitrate, econazole nitrate, tioconazole, zinc diethyldithiocarbamate, thianthol, tolciclate, tolnaftate, haloprogin, berberine benzoate, hinokitiol, chlorhexidine hydrochloride, diphenylimidazole, D-camphor, diethyl phthalate, chlorohydroxy aluminum, chitosan, turpentine oil, olive oil, mannitol, glucose, chitin, silica, hydrated silica, fructose, lactose, sucrose, saccharose, sodium stearate, aspartame, sorbitol, xylitol, stevioside, shellac, titanium oxide, sodium chloride, talc, macrogol, tannic acid, sodium copper chlorophyllin, potassium iodide, methylsiloxane, isopropyl alcohol, vitamin K, thrombin, growth factors such as EGF and FGF, and various antibiotics and immunosuppressants.

When the functional component is added to the aqueous dispersion of the present invention, the amount of the functional component added is preferably 0.01% by mass to 50% by mass with respect to the total amount of the aqueous dispersion of the present invention.

In addition to the polymers and the functional components described above, for example, a water-soluble component exhibiting another hemostatic action, for example, a hemostatic including a "blood coagulation factor preparation" such as a factor VIII preparation, a factor IX complex concentrate, and a fibrinogen preparation, a "hemostatic from organs/snake venoms preparation" such as a thromboplastin analog, lipido thromboplastin, and hemocoagulase, an "antifibrolytic agent (antiplasmin agent)" such as ε-aminocaproic acid, tranexamic acid, gabexate mesilate, and aprotinin, and "adrenaline" applied to local hemostasis (e.g., nasal bleeding) using a vasoconstriction action can be mixed in the aqueous dispersion containing insoluble polysaccharides of the present invention.

The aqueous dispersion of the present invention is capable of spraying, and the spraying can cause plasma, serum, or blood to solidify. For this reason, the aqueous dispersion of the present invention is suitably used for hemostasis and wound healing applications. The aqueous dispersion can be used for a film or a sheet.

Further, the aqueous dispersion of the present invention can be applied to an external preparation, a hemostatic composition, a wound healing composition, or an adhesion preventing composition.

### Examples

### [Production Example 1: Preparation of Aqueous Dispersion of 1.2% Microcrystalline Cellulose-Derived Nanocellulose (MNC)]

1,000 parts by mass of pure water was added to 15 parts by mass of commercially available microcrystalline cellulose (FUNACEL powder II for column chromatography available from Funakoshi Co., Ltd.) and the cellulose was dispersed. The dispersion was subjected to a pulverization treatment 300 times at 200 MPa using a high-pressure pulverization device (Star Burst system) manufactured by Sugino Machine Limited to obtain an aqueous dispersion of cellulose fibers derived from microcrystalline cellulose. The obtained dispersion was weighed and placed in a petri dish, and dried at 110°C for 5 hours to remove water. The amount of the residue was measured, and the concentration was determined. As a result, the cellulose concentration (solid content concentration) in water was 1.2% by mass, and the pH was 7. The aqueous dispersion was autoclaved at 121°C for 20 minutes.

### [Production Example 2: Preparation of Aqueous Dispersion of 1% Pulp-Derived Nanocellulose (PNC)]

978 parts by mass of pure water was added to 22 parts by mass of commercially available kraft pulp (LBKP D-8 available from Kokusai Pulp & Paper Co., Ltd., solid content: 46% by mass) and the kraft pulp was dispersed. The dispersion was subjected to a pulverization treatment 200 times at 245 MPa using a high-pressure pulverization device (Star Burst system) manufactured by Sugino Machine Limited to obtain an aqueous dispersion of cellulose fibers derived from pulp. The obtained dispersion was weighed and placed in a petri dish, and dried at 110°C for 5 hours to remove water. The amount of the residue was measured, and the concentration was determined. As a result, the cellulose concentration (solid content concentration) in water was 1% by mass, and the pH was 7. The aqueous dispersion was autoclaved at 121°C for 20 minutes.

### [Production Example 3: Preparation of 1 % Nanochitin Aqueous Dispersion]

990 parts by mass of pure water was added to 10 parts by mass of commercially available chitin powder (available from KOYO CHEMICAL CO., LTD.) and the chitin powder was dispersed. The dispersion was subjected to a pulverization treatment 200 times at 245 MPa using a high-pressure pulverization device (Star Burst system) manufactured by Sugino Machine Limited to obtain an aqueous dispersion of nanochitin fibers. The obtained dispersion was weighed and placed in a petri dish, and dried at 110°C for 5 hours to remove water. The amount of the residue was measured, and the concentration was determined. As a result, the chitin concentration (solid content concentration) in water was 1% by mass. The aqueous dispersion was autoclaved at 121°C for 20 minutes.

### [Comparative Production Example 1: Preparation of 1% Nanochitosan Aqueous Dispersion]

990 parts by mass of pure water was added to 10 parts by mass of commercially available chitosan powder (available from Sigma-Aldrich Co. LLC.) and the chitosan powder was dispersed. The dispersion was subjected to a pulverization treatment 200 times at 245 MPa using a high-pressure pulverization device (Star Burst system) manufactured by Sugino Machine Limited to obtain an aqueous dispersion of nanochitosan fibers. The obtained dispersion was weighed and placed in a petri dish, and dried at 110°C for 5 hours to remove water. The amount of the residue was measured, and the concentration was determined. As a result, the chitosan concentration (solid content concentration) in water was 1% by mass. The aqueous dispersion was autoclaved at 121°C for 20 minutes.

### [Comparative Production Example 2: Preparation of 1% CMC-Na Aqueous Solution]

0.25 g of carboxymethylcellulose sodium (CMC-Na, available from AS ONE Corporation) was placed in a 100-mL glass bottle with a lid, and 24.75 g of purified water was added to the glass bottle. The mixture was heated at 90°C for 90 minutes in a dry bath incubator, and allowed to stand at 23 °C for 24 hours at room temperature to obtain a 1% by mass CMC-Na aqueous solution.

### [Comparative Production Example 3: Preparation of 1% Tamarind Gum Aqueous Solution]

0.25 g of tamarind gum (available from Sansho Co., Ltd.) was placed in a screw tube (Maruemu No. 7), and 24.75 g of water was added and mixed at room temperature to obtain a 1% by mass tamarind gum aqueous solution.

### [Comparative Production Example 4: Preparation of 1% Xanthan Gum Aqueous Solution]

0.25 g of xanthan gum (available from Sansho Co., Ltd.) was placed in a screw tube (Maruemu No. 7), and 24.75 g of water was added and mixed at room temperature to obtain a 1% by mass tamarind gum aqueous solution.

### [Comparative Production Example 5: Preparation of 1% Gum Arabic Aqueous Solution and 5% Gum Arabic Aqueous Solution]

0.1 g of gum arabic (available from Fujii Yakuhin K.K.) was placed in a screw tube with a lid (Maruemu No. 7), and 9.9 g of purified water was added to the tube. The mixture was heated at 90°C for 60 minutes in a dry bath incubator, and allowed to stand at 23°C for 24 hours at room temperature to obtain a 1% by mass gum arabic aqueous solution.

Similarly, 0.5 g of gum arabic (available from Fujii Yakuhin K.K.) was placed in a screw tube with a lid (Maruemu No. 7), and 9.5 g of purified water was added to the tube. The mixture was heated at 90°C for 60 minutes in a dry bath incubator, and allowed to stand at 23°C for 24 hours at room temperature to obtain a 5% by mass gum arabic aqueous solution.

### [Comparative Production Example 6: Preparation of 1% Sodium Alginate Aqueous Solution]

0.1 g of sodium alginate (available from Hayashi Chemical) was placed in a screw tube with a lid (Maruemu No. 7), and 9.9 g of purified water was added to the tube. The mixture was heated at 90°C for 60 minutes in a dry bath incubator, and allowed to stand at 23°C for 24 hours at room temperature to obtain a 1% by mass sodium alginate aqueous solution.

### [Comparative Production Example 7: Preparation of 1% Deacylated Gellan Gum Aqueous Solution]

0.25 g of deacylated gellan gum (available from Sansho Co., Ltd.) was placed in a screw tube (Maruemu No. 7), and 17.5 g of water was added to the tube. The mixture was heated at 90°C for 60 minutes in a dry bath incubator, and allowed to stand at 23°C for 24 hours at room temperature to obtain a 1% by mass deacylated gellan gum aqueous solution.

### [Comparative Production Example 8: Preparation of 1% PVA Aqueous Solution]

0.1 g of polyvinyl alcohol (PVA) (JF-17, available from JAPAN VAM & POVAL CO., LTD.) was placed in a screw tube with a lid (Maruemu No. 7), and 9.9 g of purified water was added to the tube. The mixture was heated at 90°C for 60 minutes in a dry bath incubator, and allowed to stand at 23°C for 24 hours at room temperature to obtain a 1% by mass PVA aqueous solution.

### [Measurements of Average Fiber Diameter D and Average Fiber Length L]

In accordance with the procedure described in [0022], the average fiber diameter D and the average fiber length L of the cellulose fibers, the nanochitin fibers, and the nanochitosan fibers obtained in Production Examples 1 to 3 and Comparative Production Example 1 were measured from a TEM image and a SEM image. The aspect ratio L/D was determined from the average fiber diameter D and the average fiber length L. The obtained results are shown in Table 1.

**Table 1**

| | Average Fiber Diameter D [nm] | Average Fiber Length L (nm) | Aspect Ratio L/D |
|---|---|---|---|
| Production Example 1 (MNC) | 24 | 594 | 24 |
| Production Example 2 (PNC) | 19 | 1960 | 103 |
| Production Example 3 (Nanochitin) | 12 | 352 | 29 |
| Comparative Production Example 1 (Nanochitosan) | 35 | 2525 | 72 |

### [Example 1: Solidifying Action of 1.2% MNC Aqueous Dispersion and 1% PNC Aqueous Dispersion on Human Serum]

0.5 mL of human serum (A-type, available from KAC Co., Ltd.) was placed in a plastic tube, and 0.5 mL of the 1.2% microcrystalline cellulose aqueous dispersion was added to the tube. Immediately after mixing, the solidifying action on the serum was confirmed, and at this time, slight syneresis was confirmed. To the mixture, 0.5 mL of the 1.2% microcrystalline cellulose aqueous dispersion was further added and mixed. The serum completely solidified without syneresis.

0.5 mL of human serum (A-type, available from KAC Co., Ltd.) was placed in a plastic tube, and 0.5 mL of the 1% pulp-derived cellulose aqueous dispersion was added to the tube. Immediately after mixing, the solidifying action on the serum was confirmed, and at this time, the formation of sol due to a part of syneresis was confirmed. To the mixture, 0.5 mL of the 1% pulp-derived cellulose aqueous dispersion was further added and mixed. The serum completely solidified without syneresis.

As the human serum, frozen serum (A type) available from KAC Co., Ltd., was melted at 4°C for 20 hours, and used in a test.

### [Example 2: Solidifying Action of 1% PNC Aqueous Dispersion on Human Plasma]

1 mL of human plasma (available from Kohjin Bio Co., Ltd., treated with heparin sodium) was placed in a plastic tube, and 1 mL of the 1% pulp-derived cellulose aqueous dispersion was added to the tube. Immediately after mixing, the thickening and solidifying action on the plasma was confirmed.

1 mL of human plasma (available from Kohjin Bio Co., Ltd., treated with heparin sodium) was placed in another plastic tube, and 2 mL of the 1% pulp-derived cellulose aqueous dispersion was added to the tube. Immediately after mixing, the solidifying action on the plasma was confirmed.

As the human plasma, frozen plasma available from Kohjin Bio Co., Ltd., was melted at room temperature for 5 hours, and used in a test.

### [Example 3: Solidifying Action of 1.2% MNC Aqueous Dispersion and 1% PNC Aqueous Dispersion on Human Blood]

0.5 mL of human blood (available from KAC Co., Ltd.) was placed in a plastic tube, and 0.5 mL of the 1.2% microcrystalline cellulose aqueous dispersion was added to the tube. Immediately after mixing, the solidifying action on the blood was confirmed. To the mixture, 0.5 mL of aqueous dispersion of the 1.2% microcrystalline cellulose was further added. The solidification state of the blood was maintained.

0.5 mL of human blood (available from KAC Co., Ltd.) was placed in a plastic tube, and 0.5 mL of the 1% pulp-derived cellulose aqueous dispersion was added to the tube. Immediately after mixing, the solidifying action on the blood was confirmed, and at this time, partial syneresis was confirmed. To the mixture, 0.5 mL of the 1% pulp-derived cellulose aqueous dispersion was further added and mixed. The blood completely solidified without syneresis.

As the human blood, frozen blood available from KAC Co., Ltd. (treated with heparin sodium) was melted at 4°C for 20 hours, and used in a test.

### [Example 4: Solidifying Action of 1.2% MNC Aqueous Dispersion and 1% PNC Aqueous Dispersion on Rabbit Blood]

1 mL of rabbit blood was placed in a plastic tube, and 0.5 mL or 1 mL of the 1.2% microcrystalline cellulose aqueous dispersion was added to the tube. Immediately after mixing, the solidifying action on the blood was confirmed for both the addition amounts.

To 1 mL of rabbit blood, 0.5 mL of the 1% pulp-derived cellulose aqueous dispersion was added. Immediately after mixing, the solidifying action on the blood was confirmed. This sample was observed with an optical microscope. It was confirmed that blood cells in the blood were incorporated into a network of the cellulose fibers. FIG. 1 shows an optical micrograph at that time.

As the rabbit blood, blood was collected from the abdominal aorta of rabbit (available from Japan SLC, Inc.) by a plastic injection syringe using a 3.8% sodium citrate solution (blood: sodium citrate solution = 9:1), placed in a plastic tube, and used in a test.

### [Example 5: Solidifying Action of 1% PNC Aqueous Dispersion By Spray Mist Method on Bovine Plasma]

0.5 mL of bovine serum (available from Life Technologies Japan Ltd.) was placed in a plastic petri dish. The 1% pulp-derived cellulose was placed in a spray bottle (Maruemu 3 L), and sprayed in an amount of 1 mL on the bovine serum (added in a mist form). Immediately after contact between the bovine serum and cellulose in the mist form, the fluidity of the bovine serum was lost in the petri dish. The solidifying of the serum was confirmed.

### [Example 6: Solidifying Action of 1% Chitin Aqueous Dispersion on Human Serum and Blood]

0.5 mL of human serum (A-type, available from KAC Co., Ltd.) was placed in a plastic tube, and 0.5 mL of the 1% chitin aqueous dispersion was added to the tube. Immediately after mixing, the solidifying action on the serum was confirmed, and at this time, slight syneresis was confirmed. To the mixture, 0.5 mL of the 1% chitin aqueous dispersion was further added and mixed. The serum completely solidified without syneresis.

0.5 mL of human blood (available from KAC Co., Ltd.) was placed in a plastic tube, and 0.5 mL of the 1% chitin aqueous dispersion was added to the tube. Immediately after mixing, the solidifying action on the blood was confirmed, and syneresis was confirmed. To the mixture, 0.5 mL of the 1% chitin aqueous dispersion was further added and mixed. The blood solidified.

### [Comparative Example 1: Solidifying Action of 1% PuraMatrix on Human Serum and Blood]

0.5 mL of human serum (A-type, available from KAC Co., Ltd.) was placed in a plastic tube, and 0.5 mL of PuraMatrix (registered trademark) hydrogel (available from BD Biosciences) containing 1%w/v polymer peptide as an active ingredient was added to the tube. Immediately after mixing, the solidifying action on the serum was confirmed, and at this time, syneresis was confirmed. To the mixture, 0.5 mL of PuraMatrix was further added and mixed. The serum completely solidified without syneresis.

0.5 mL of human blood (available from KAC Co., Ltd.) was placed in a plastic tube, and 0.5 mL of PuraMatrix described above was added to the tube. Immediately after mixing, the solidifying action on the blood was confirmed, and syneresis was confirmed. To the mixture, 0.5 mL of PuraMatrix was further added and mixed. The blood completely solidified without syneresis.

### [Comparative Example 2: Solidifying Action of 1% Chitosan Aqueous Dispersion on Human Serum and Human Blood]

0.5 mL of human serum (A-type, available from KAC Co., Ltd.) was placed in a plastic tube, and 0.5 mL of the 1% chitosan aqueous dispersion was added to the tube. The serum did not solidify and was in a liquid state. To the mixture, 0.5 mL of the 1% chitosan aqueous dispersion was further added and mixed. The solidifying of the serum was not confirmed.

0.5 mL of human blood (available from KAC Co., Ltd.) was placed in a plastic tube, and 0.5 mL of the 1% chitosan aqueous dispersion was added and mixed. The solidifying action on the blood was not confirmed. To the mixture, 0.5 mL of the 1% chitosan aqueous dispersion was further added and mixed. The solidifying action on the blood was not confirmed.

### [Comparative Example 3: Solidifying Action of 1% CMC-Na Aqueous Solution on Human Serum]

0.5 mL of human serum (A-type, available from KAC Co., Ltd.) was placed in a plastic tube, and 0.5 mL of the 1% CMC-Na aqueous solution was added and mixed. The serum did not solidify and was in a liquid state. To the mixture, 0.5 mL of the 1% CMC-Na aqueous solution was further added and mixed. The solidifying of the serum was not confirmed.

### [Comparative Example 4: Solidifying Action of 1% Tamarind Gum Aqueous Solution on Human Serum]

0.5 mL of human serum (A-type, available from KAC Co., Ltd.) was placed in a plastic tube, and 0.5 mL of the 1% tamarind gum aqueous solution was added and mixed. The serum did not solidify and was in a liquid state. To the mixture, 0.5 mL of the 1% tamarind gum aqueous solution was further added and mixed. The solidifying of the serum was not confirmed.

### [Comparative Example 5: Solidifying Action of 1% Xanthan Gum Aqueous Solution on Human Serum]

0.5 mL of human serum (A-type, available from KAC Co., Ltd.) was placed in a plastic tube, and 0.5 mL of the 1% xanthan gum aqueous solution was added and mixed. The serum did not solidify and was in a liquid state. To the mixture, 0.5 mL of the 1% xanthan gum aqueous solution was further added and mixed. The solidifying of the serum was not confirmed.

### [Comparative Example 6: Solidifying Action of 1% or 5% Gum Arabic Aqueous Solution on Human Serum, Blood, and Plasma]

0.5 mL of human serum (A-type, available from KAC Co., Ltd.) was placed in a plastic tube, and 0.5 mL of the 1% gum arabic aqueous solution was added and mixed. The serum did not solidify and was in a liquid state. To the mixture, 0.5 mL of the 1% gum arabic aqueous solution was further added and mixed. The solidifying of the serum was not confirmed.

Similarly, 0.5 mL of human serum (A-type, available from KAC Co., Ltd.) was placed in a plastic tube, and 0.5 mL of the 5% gum arabic aqueous solution was added and mixed. The serum did not solidify and was in a liquid state. To the mixture, 0.5 mL of the 5% gum arabic aqueous solution was further added and mixed. The solidifying of the serum was not confirmed.

0.5 mL of human blood (available from KAC Co., Ltd.) was placed in a plastic tube, and 0.5 mL of the 1% gum arabic aqueous solution was added and mixed. The blood did not solidify and was in a liquid state. To the mixture, 0.5 mL of the 1% gum arabic aqueous solution was further added. The solidifying of the blood was not confirmed.

Similarly, 0.5 mL of human blood (available from KAC Co., Ltd.) was placed in a plastic tube, and 0.5 mL of the 5% gum arabic aqueous solution was added and mixed. The blood did not solidify and was in a liquid state. To the mixture, 0.5 mL of the 5% gum arabic aqueous solution was further added and mixed. The solidifying of the blood was not confirmed.

Further, 0.5 mL of human plasma (available from Kohjin Bio Co., Ltd.) was placed in a plastic tube, and 0.5 mL of the 1% gum arabic aqueous solution was added and mixed. The plasma did not solidify and was in a liquid state. To the mixture, 0.5 mL of the 1% gum arabic aqueous solution was further added and mixed. The solidifying of the plasma was not confirmed.

Similarly, 1 mL of human plasma (available from Kohjin Bio Co., Ltd.) was placed in a plastic tube, and 1 mL of the 5% gum arabic aqueous solution was added and mixed. The plasma did not solidify and was in a liquid state. To the mixture, 2 mL of the 5% gum arabic aqueous solution was further added. The solidifying of the plasma was not confirmed.

### [Comparative Example 7: Solidifying Action of 1% Sodium Alginate Aqueous Solution on Human Serum, Blood, and Plasma]

0.5 mL of human serum (A-type, available from KAC Co., Ltd.) was placed in a plastic tube, and 0.5 mL of the 1% sodium alginate aqueous solution was added and mixed. The serum did not solidify and was in a liquid state. To the mixture, 0.5 mL of the 1% sodium alginate aqueous solution was further added and mixed. The solidifying of the serum was not confirmed.

Similarly, 0.5 mL of human blood (available from KAC Co., Ltd.) was placed in a plastic tube, and 0.5 mL of the 1% sodium alginate aqueous solution was added and mixed. The blood did not solidify and was in a liquid state. To the mixture, 0.5 mL of the 1% sodium alginate aqueous solution was further added and mixed. The solidifying of the blood was not confirmed.

Further, 0.5 mL of human plasma (available from Kohjin Bio Co., Ltd.) was placed in a plastic tube, and 1 mL of the 1% sodium alginate aqueous solution was added and mixed. The plasma did not solidify and was in a liquid state. To the mixture, 2 mL of 1% sodium alginate aqueous solution was further added and mixed. The solidifying of the plasma was not confirmed.

### [Comparative Example 8: Solidifying Action of 1% Deacylated Gellan Gum Aqueous Solution on Human Serum]

0.5 mL of human serum (A-type, available from KAC Co., Ltd.) was placed in a plastic tube, and 0.5 mL of the 1% deacylated gellan gum aqueous solution was added and mixed. The serum did not solidify and was in a liquid state. To the mixture, 0.5 mL of the 1% deacylated gellan gum aqueous solution was further added and mixed. The solidifying of the serum was not confirmed.

The present inventors have confirm that when in an aqueous dispersion containing cellulose fibers, the fiber diameter is 0.001 to 0.05 µm and the aspect ratio is 5 to 500, the aqueous dispersion can be sprayed without dripping (Japanese Patent Application No. 2012-265530 (JP 2012-265530 A)). It has been confirmed that an aqueous dispersion of pulp-derived cellulose exhibits a thickening action on various electrolytes, but an aqueous dispersion of microcrystalline cellulose does not exhibit a thickening action on a salt (Japanese Patent Application No. 2013-89702 (JP 2013-89702 A)).

In Examples of the present invention, it was confirmed that the aqueous dispersion of microcrystalline cellulose caused serum and blood to solidify (Examples 1 and 3), and the aqueous dispersion of pulp-derived cellulose caused serum, plasma, and blood to solidify (Examples 1 to 3). Therefore, it is estimated that these nanocelluloses interact with various ingredients in blood, serum, or plasma to form an agglomerate or an assembly of nanofibers. Consequently, it is considered that the aqueous dispersion containing cellulose fibers causes serum or plasma itself to solidify, and as a result, blood solidify to exhibit a hemostatic action.

With respect to blood, further investigation was performed. The solidifying action on human blood (0.5 mL) was confirmed using the 1.2% microcrystalline cellulose aqueous dispersion in a small amount of 0.5 mL. However, even using the 1% pulp-derived cellulose aqueous dispersion, the 1% chitin aqueous dispersion, or PuraMatrix in each addition amount of 0.5 mL, partial formation of sol due to syneresis was found.

The obtained results are shown in Tables 2 and 3.

**Table 2**

| Aqueous dispersion/Aqueous solution, Addition amount | Human serum (0.5 mL) | | Human blood (0.5 mL) | |
|---|---|---|---|---|
| | 0.5 mL addition | 1 mL addition | 0.5 mL addition | 1 mL addition |
| 1.2% MNC | Δ | ○ | ○ | ○ |
| 1% PNC | Δ | ○ | Δ | ○ |
| 1% chitin | Δ | ○ | Δ | ○ |
| 1% PuraMatrix | Δ | ○ | Δ | ○ |
| 1% chitosan | × | × | × | × |
| 1% CMC-Na | × | × | | - |
| 1% tamarind gum | × | × | - | - |
| 1% xanthan gum | × | × | - | - |
| 1% gum arabic | × | × | × | × |
| 5% gum arabic | × | × | × | × |
| 1% sodium alginate | × | × | × | × |
| 1% deacylated gellan gum | × | × | - | - |

| | | | | |
|---|---|---|---|---|
| *MNC: microcrystalline cellulose *PNC: pulp-derived cellulose ○: A subject completely solidified without syneresis (within 20 seconds immediately after contact) Δ: Slight syneresis was confirmed, and a solidifying action was confirmed X: A solidifying action was not confirmed | | | | |

**Table 3**

| Aqueous dispersion/Aqueous solution, Addition amount | Human plasma (0.5 mL) | | | Human plasma (1 mL) | | |
|---|---|---|---|---|---|---|
| | 0.5 mL addition | 1 mL addition | 3 mL addition | 0.5 mL addition | 1 mL addition | 3 mL addition |
| 1 % PNC | - | - | - | Δ | ○ | - |
| 1% gum arabic | × | × | - | - | - | - |
| 5% gum arabic | - | - | - | × | - | × |
| 1% sodium alginate | - | × | × | - | - | - |

| | | | | | | |
|---|---|---|---|---|---|---|
| ○: A subject completely solidified without syneresis (within 20 seconds immediately after contact) Δ: A thickening and solidifying action was confirmed X: A solidifying action was not confirmed | | | | | | |

### [Example 7: Observation of Hydrophobicity with Fluorescent Microscope]

To 1 mL of each of the 1% pulp-derived cellulose aqueous dispersion, the 1% deacylated gellan gum aqueous solution, the 1% nanochitin aqueous dispersion, and the 1% nanochitosan aqueous dispersion, 0.007 g of anilinonaphthalenesulfonic acid (ANS) was added and dissolved. Droplets of each of the aqueous dispersions and the aqueous solution were placed on a preparation, dried at room temperature, and observed with a fluorescent microscope.

To 0.5 mL of each of the 1% pulp-derived cellulose aqueous dispersion and the 1% nanochitin aqueous dispersion, 0.005 mL of 10% NaCl solution was added, 0.007 g of ANS was then added and dissolved. Observation with a fluorescent microscope was similarly performed.

The observation results are shown below.

Anilinonaphthalenesulfonic acid is known to have significantly increased fluorescence intensity under a hydrophobic environment as compared with in pure water, and to have decreased fluorescence intensity (or not to express fluorescence) under a hydrophilic environment.

To the 1% pulp-derived cellulose dispersion in which the solidifying action on serum, plasma, and blood was confirmed in Examples described above, anilinonaphthalenesulfonic acid (ANS) was added. Fluorescence was not confirmed. Therefore, the environment was hydrophilic. On the other hand, to the 1% pulp-derived cellulose dispersion, a NaCl solution was added, and ANS was further added. Fluorescence was confirmed, and therefore change into a hydrophobic environment (hydrophobic structure) was confirmed.

As considered from the aforementioned results, cellulose is usually water-insoluble polysaccharides, but cellulose that is made finer into nanofibers has a hydrophilic fiber surface and has a mechanism in which the fiber surface becomes hydrophobic by adsorption of a salt or the like on the fiber surface.

The micronized cellulose fibers that are a component of the cellulose dispersion as described above express hydrophobicity by hydrophilicity/hydrophobicity change on the surface, specifically, by an action of a sodium salt or the like contained in serum or the like on the surface of the cellulose nanofibers, and are subjected to a complex-forming reaction with an ingredient in plasma, serum, or blood, that is, the micronized cellulose and the ingredient in serum, plasma, or the like form an agglomerate or an assembly. Thus, the cellulose fibers may express the solidifying action on plasma, serum, or blood.

To the 1% deacylated gellan gum aqueous solution, anilinonaphthalenesulfonic acid was added. An increase in fluorescence intensity was confirmed.

Deacylated gellan gum generally has a property in which it is self-assembled by a reaction with calcium ions to form a gel. However, the deacylated gellan gum is affected by a reaction with an ionic substance in serum or blood in terms of properties of chemical structure, and as a result, the reaction with calcium ions is inhibited. For this reason, the deacylated gellan gum and the ingredient in serum or blood do not form a complex, that is, an agglomerate/assembly containing the deacylated gellan gum and serum or the like is not formed. Therefore, it is considered that the solidifying action of the deacylated gellan gum on serum or blood is confirmed.

To each of the 1% nanochitin aqueous dispersion and the 1% nanochitosan aqueous dispersion, anilinonaphthalenesulfonic acid was added. For both the dispersions, fluorescence was confirmed. Since chitosan is known to be largely affected by ions, an agglomerate/assembly is not formed, that is, serum and blood do not solidify. On the other hand, since chitin is not affected by ions, only chitin is reacted with the ingredient in serum or blood to form a complex. As a result, an agglomerate/an assembly is formed. Therefore, expression of solidifying action on serum and blood is predicted.

### [Example 8: Composition of Nanocellulose and Polymer]

To 9 g of the 1% pulp-derived cellulose dispersion, 1 g of 10% PVA aqueous solution was added to prepare a transparent dispersion containing 0.9% by mass cellulose and 1% by mass PVA. The transparent dispersion containing 0.9% by mass cellulose and 1% by mass PVA was placed in a spray bottle (Maruemu 3 L), and spraying was performed using this bottle. Since the transparent dispersion was converted into a mist form, spraying was possible. 5 g of the transparent dispersion containing 0.9% by mass cellulose and 1% by mass PVA was sprayed, and dried at room temperature for 48 hours. The sprayed area had a film form.

### [Example 9: Formation f Water-Containing Sheet of Nanocellulose]

To 9 g of the 1% pulp-derived cellulose dispersion, 1 g of 10% PVA aqueous solution was added to prepare a transparent dispersion containing 0.9% by mass cellulose and 1% by mass PVA. 10 g of the transparent dispersion containing 0.9% by mass cellulose and 1% by mass PVA was placed in a plastic case (5 cm × 8 cm), and air-dried at room temperature to obtain 0.171 g of transparent film. This film was placed in a plastic petri dish (diameter: 6 cm), and 10 mL of water was added. Water was instantly absorbed on the film to produce a water-containing sheet.

### [Example 10: Investigation of Blood Coagulation and Fibrinolysis Systems]

For investigation of blood coagulation using Data-Fi fibrinogen (available from SYSMEX COROPORATION) in a fully automated blood coagulation analyzer CS-200i (manufactured by SYSMEX COROPORATION), 50 µL of thrombin reagent was added to 10 µL of human standard plasma Ci-Trol, and 10 µL of the 1.2% microcrystalline cellulose aqueous dispersion was then added. Formation of fibrin was not confirmed.

For investigation of fibrinolysis system using fibrin/fibrinogen degradation product measurement reagent Latextest BL-2 P-FDP (available from SYSMEX COROPORATION) in a fully automated blood coagulation analyzer CS-200i (manufactured by SYSMEX COROPORATION), 10 µL of aqueous dispersion of human standard serum FDP control neo H and 1.2% microcrystalline cellulose was investigated. Formation of fibrin/fibrinogen degradation product was not confirmed.

### [Production Example 4: Preparation of Aqueous Dispersion of 1.7% Pulp-Derived Nanocellulose (PNC)]

128 parts by mass of pure water was added to 22 parts by mass of kraft pulp (LBKP D-8 available from Hokuetsu Kishu Paper Co., Ltd., solid content: 35% by mass) resulting in dispersion, and a pulverization treatment was performed 9 times at 1,500 rpm using a millstone type grinding device (Super Masscolloider) manufactured by MASUKO SANGYO CO., LTD., to prepare a pulp slurry (solid content concentration: 3.5% by mass). This pulp slurry was subjected to a pulverization treatment 300 times at 245 MPa using a high-pressure pulverization device (Star Burst system) manufactured by Sugino Machine Limited to obtain an aqueous dispersion of cellulose fibers derived from pulp. The obtained dispersion was weighed and placed in a petri dish, and dried at 110°C for 5 hours to remove water. The amount of the residue was measured, and the concentration was determined. As a result, the cellulose concentration (solid content concentration) in water was 1.7% by mass. The aqueous dispersion was autoclaved at 121°C for 20 minutes.

### [Example 11: Composition of Nanocellulose and Functional Component (Benzalkonium Chloride)]

9.5 g of water was added to 0.5 g of benzalkonium chloride (50%) to prepare a 2.5% benzalkonium chloride aqueous solution.

0.25 mL of the 2.5% benzalkonium chloride aqueous solution was placed in a plastic tube, and 2 mL of the 1.7% pulp-derived nanocellulose aqueous dispersion was added to the tube and mixed. At that time, gelation (solidification) was confirmed.

Similarly, 0.5 mL of the 2.5% benzalkonium chloride aqueous solution was placed in a plastic tube, and 2 mL of the 1.7% pulp-derived nanocellulose aqueous dispersion was added and mixed. At that time, gelation (solidification) was confirmed. 1 mL of the 2.5% benzalkonium chloride aqueous solution was placed in a plastic tube, and 2 mL of the 1.7% pulp-derived nanocellulose aqueous dispersion was added to the tube and mixed. At that time, gelation (solidification) was confirmed.

The obtained results are shown in Table 4.

**Table 4**

| | | | |
|---|---|---|---|
| 1.7% Pulp-derived nanocellulose aqueous dispersion | 2 mL | 2 mL | 2 mL |
| 2.5% Benzalkonium chloride aqueous solution | 0.25 mL | 0.5 mL | 1 mL |
| State of mixed solution | Gel | Gel | Gel |

### [Example 12: Composition of Nanocellulose and Functional Component (Aminocaproic Acid)]

9.8 g of water was added to 0.2 g of aminocaproic acid (6-aminohexanoic acid) to prepare a 2% aminocaproic acid aqueous solution.

0.25 mL of the 2% aminocaproic acid aqueous solution was placed in a plastic tube, and 2 mL of the 1.7% pulp-derived nanocellulose aqueous dispersion was added to the tube and mixed. At that time, gelation (solidification) was confirmed.

Similarly, 0.5 mL of the 2% aminocaproic acid aqueous solution was placed in a plastic tube, and 2 mL of the 1.7% pulp-derived nanocellulose aqueous dispersion was added to the tube and mixed. At that time, gelation (solidification) was confirmed.

The obtained results are shown in Table 5.

**Table 5**

| | | |
|---|---|---|
| 1.7% Pulp-derived nanocellulose aqueous dispersion | 2 mL | 2 mL |
| 2.5% Aminocaproic acid aqueous solution | 0.25 mL | 0.5 mL |
| State of mixed solution | Gel | Gel |

### [Production Example 5: Preparation of Aqueous Dispersion of 1% Microcrystalline Cellulose-derived Nanocellulose (MNC)]

396 parts by mass of pure water was added to 4 parts by mass of commercially available crystalline cellulose (PH-101 available from Asahi Kasei Chemicals Corporation), resulting in dispersion. The aqueous dispersion was produced by the same operation as in Production Example 1. The cellulose concentration (solid content concentration) in water was 1.0% by mass.

### [Production Example 6: Preparation of 2% Chitin Aqueous Dispersion]

Commercially available biomass nanofibers (2% by mass of BiNFi-S, available from Sugino Machine Limited) was autoclaved at 121°C for 20 minutes.

### [Reference Example 1: State of Blood in Mixing Water in Heparin-Treated Blood of Rat]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 10 mL of blood was collected from the abdominal aorta using a plastic syringe with an 18-G needle having a top coated with heparin (1,000 U/mL, SAGENT).

1 mL of the heparin-treated blood of the rat was placed in a plastic tube, and 1 mL of water was added to the tube. The mixture was in a liquid state. 1 mL of water was further added. The mixture was in a liquid state.

### [Example 13: Solidifying action of Aqueous Dispersion of 1% Microcrystalline Cellulose-derived Nanocellulose (MNC) on Heparin-Treated Blood]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 10 mL of blood was collected from the abdominal aorta using a plastic syringe with an 18-G needle having a top coated with heparin (1,000 U/mL, SAGENT).

1 mL of the heparin-treated blood of the rat was placed in a plastic tube, and 1 mL of 1% microcrystalline cellulose-derived nanocellulose aqueous dispersion was added to the tube. The mixture was in a liquid state. 1 mL of the 1% microcrystalline cellulose-derived nanocellulose aqueous dispersion was further added. Immediately after the addition, the solidifying action on blood was confirmed, and at this time, syneresis was confirmed.

1 mL of this blood was placed in a plastic tube, and 1.5 mL of the 1% microcrystalline cellulose-derived nanocellulose aqueous dispersion was added to the tube. This blood is in a syneresis state, but a solidifying tendency was confirmed.

### [Example 14: Solidifying Action of Heparin-Treated Blood by 2% Chitin Aqueous Dispersion]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 10 mL of blood was collected from the abdominal aorta using a plastic syringe with an 18-G needle having a top coated with heparin (1,000 U/mL, SAGENT).

1 mL of the heparin-treated blood of the rat was placed in a plastic tube, and 1 mL of the 2% chitin aqueous dispersion was added to the tube. Immediately after mixing, the solidifying action of blood was confirmed, and at this time, slight syneresis was confirmed. To the mixture, 1 mL of the 2% chitin aqueous dispersion was further added and mixed. Blood solidified without syneresis.

1 mL of this blood was placed in a plastic tube, and 1.5 mL of the 2% chitin aqueous dispersion was added. The blood solidified without syneresis.

### [Example 15: Solidifying Action of 1.7% Pulp-Derived Nanocellulose Aqueous Dispersion on Heparin-Treated Blood]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 10 mL of blood was collected from the abdominal aorta using a plastic syringe with an 18-G needle having a top coated with heparin (1,000 U/mL, SAGENT).

1 mL of the heparin-treated blood of the rat was placed in a plastic tube, and 1 mL of the 1.7% pulp-derived nanocellulose aqueous dispersion was added to the tube. Immediately after mixing, the solidifying action of blood was confirmed, and at this time, slight syneresis was confirmed. To the mixture, 1 mL of the 1.7% pulp-derived cellulose aqueous dispersion was further added and mixed. The blood solidified without syneresis.

1 mL of this blood was placed in a plastic tube, and 1.5 mL of the 1.7% pulp-derived nanocellulose aqueous dispersion was added. The blood solidified without syneresis.

### [Reference Example 2: State of Blood in Mixing Water with Blood of Rat]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 10 mL of blood was collected from the abdominal aorta using a plastic syringe with an 18-G needle.

1.5 mL of the blood of the rat was placed in a plastic tube, and 1 mL of water was added to the tube. The mixture was in a liquid state. After a dispersion treatment (15 seconds) using a vortex mixer, the mixture was in a liquid state.

### [Example 16: Solidifying Action on Blood of Rat by 2% Chitin Aqueous Dispersion]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 4 mL of blood was collected from the abdominal aorta using a plastic syringe with an 18-G needle.

1 mL of the blood of the rat was placed in a plastic tube, and 1.5 mL of the 2% chitin aqueous dispersion was added to the tube. Immediately after mixing, the solidifying action on blood was confirmed, and at this time, slight syneresis was confirmed.

### [Example 17: Solidifying Action of 1.7% Pulp-Derived Nanocellulose Aqueous Dispersion on Rat Blood]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. 4 mL of blood was collected from the abdominal aorta using a plastic syringe with an 18-G needle.

1 mL of the rat blood was placed in a plastic tube, and 1.5 mL of the 1.7% pulp-derived nanocellulose aqueous dispersion was added to the tube. Immediately after mixing, the solidifying action on the blood was confirmed, and at this time, the blood solidified without syneresis. Further, even after the solidified body was subjected to a dispersion treatment (15 seconds) using a vortex mixer, syneresis was not confirmed.

### [Example 18: Blood Solidification Test using Leg Vein of Rat]

A male SD rat of 10-week-old (available from CIEA, JAC Inc.) was intraperitoneally administered with 40 mg/kg of pentobarbital sodium (available from Kyoritsu Seiyaku Corporation), and put under anesthesia. From the leg vein of the rat, 1/3 of the blood vessel was resected with a sharp scalpel, resulting in bleeding. Immediately after the bleeding, the wounded area was completely covered using a plastic syringe that was charged separately with 0.5 mL of the 1.7% pulp-derived nanocellulose (PNC) aqueous dispersion, 0.5 mL of the 2% chitin aqueous dispersion, or 0.5 mL of water. After one minute, the materials were wiped with a gauze. A state of flowing of blood from the wound and the presence or absence of infiltration into the bedded gauze were philosophically observed.

By a covering treatment with pulp-derived nanocellulose (PNC), the flowing of blood was stopped. By a covering treatment with chitin, the flowing of blood was not stopped. When water was applied, blood flowed, and the flowing was not stopped.

### [Example 19: Test of Effect of Preventing Hemostasis and Adhesion in Rat Hepatic Resection Model]

### (1) Test Method

A male rat (245.6 g to 260.9 g) was intraperitoneally administered with pentobarbital sodium (trade name: Somnopentyl, containing 64.8 mg/mL of pentobarbital sodium, available from Kyoritsu Seiyaku Corporation). Specifically, Somnopentyl was diluted 10 times with a physiological saline (serial number: K3I85, OTSUKA NORMAL SALINE, available from Otsuka Pharmaceutical Factory, Inc.). The dosage was calculated such that the dosage of pentobarbital sodium was 40 mg/kg, and administered.

Under deep anesthesia, the abdominal skin of the rat was shaved with an electric clipper, the surgical field was disinfected with rubbing alcohol, and vertical midline incision was made in the skin to expose the liver. The external left lobe of the liver was placed on a sterilized board, and 30 mm of the peripheral region was resected. In a test substance-coating group, 1g to 5 g of 1.77% pulp-derived nanocellulose was applied to the hepatic resection surface so that the resection surface was completely covered. The abdomen was sutured and closed. In an untreated group, the abdomen was sutured and closed immediately after the hepatic resection.

While the rat was sufficiently warmed, the state of the rat was observed until awakening. After the awakening, the rat was housed in a home cage.

On the seventh day after the surgery, the rat was slaughtered under anesthesia with isoflurane (ISOFLU (registered trademark) available from DS Pharma Animal Health Co., Ltd.). The adhesion of the intraperitoneal tissues and the hemorrhagic change were scored in four stages of "0: no adhesion, 1: detachable by its weight, 2: bluntly detachable, and 3: sharp detachment."

### (2) Test Results

On the surgery day, in all the subjects of the untreated group, bleeding by hepatic resection was confirmed. In the pulp-derived nanocellulose administration group in which the pulp-derived nanocellulose was applied to the hepatic resection surface, bleeding was stopped to achieve hemostasis.

For the four-stage score shown in Table as the degree of adhesion on the seventh day after the surgery, the hepatic resections of three subjects in five subjects of the untreated group exhibited a score of 3. In contrast, all subjects of the pulp-derived nanocellulose administration group exhibited a score of 0, and the effect of preventing adhesion by the hepatic resection was confirmed.

The obtained results are shown in Table 6.

**Table 6**

| Results of test of effect of preventing hemostasis and adhesion in rat hepatic resection model | | | | |
|---|---|---|---|---|
| | Animals | Test substance coating amount/g | Bleeding | Adhesion score |
| Untreated group | 1 | 0 | Bleeding | 3 |
| | 2 | 0 | Bleeding | 3 |
| | 3 | 0 | Bleeding | 0 |
| | 4 | 0 | Bleeding | 3 |
| | 5 | 0 | Bleeding | 0 |
| Pulp-derived nanocellulose group | 1 | 2.7 | No bleeding | 0 |
| | 2 | 1.8 | No bleeding | 0 |
| | 3 | 2.5 | No bleeding | 0 |
| | 4 | 3.0 | No bleeding | 0 |
| | 5 | 2.5 | No bleeding | 0 |

## Claims

1. An aqueous dispersion comprising insoluble polysaccharides having an average fiber diameter (D) of 0.001 to 100 µm and a ratio (L/D) of an average fiber length (L) to an average fiber diameter (D) of 5 to 500, **characterized in that** the aqueous dispersion causes plasma, serum, or blood to solidify under contact with plasma, serum, or blood.

2. The aqueous dispersion according to claim 1, wherein the insoluble polysaccharides are selected from the group consisting of cellulose fibers and chitin fibers, lignin, hemicellulose, inulin, protopectin, glucan, glucomannan, cyclodextrin, and dietary fibers.

3. The aqueous dispersion according to claim 1 or 2, wherein the insoluble polysaccharides are contained in a concentration of 0.001% by mass to 10% by mass.

4. The aqueous dispersion according to any one of claims 1 to 3, further comprising a polymer.

5. The aqueous dispersion according to claim 4, wherein the polymer is selected from the group consisting of collagen, alginic acid, polyvinyl alcohol, carboxymethyl cellulose, methyl cellulose, and hyaluronic acid.

6. The aqueous dispersion according to any one of claims 1 to 5, further comprising a functional component.

7. The aqueous dispersion according to claim 6, wherein the functional component is selected from the group consisting of ascorbic acid, aminocaproic acid, tranexamic acid, and thrombin.

8. The aqueous dispersion according to any one of claims 1 to 7, wherein the aqueous dispersion is capable of spraying, and the spraying causes plasma, serum, or blood to solidify.

9. Use of the aqueous dispersion according to any one of claims 1 to 8 in hemostasis application.

10. Use of the aqueous dispersion according to any one of claims 1 to 8 in wound healing application.

11. Use of the aqueous dispersion according to any one of claims 1 to 8 in adhesion preventing application.

12. A film or a sheet obtained from the aqueous dispersion according to any one of claims 1 to 8.

13. An external composition comprising the aqueous dispersion according to any one of claims 1 to 8.

14. A hemostatic composition comprising the aqueous dispersion according to any one of claims 1 to 8.

15. A wound healing composition comprising the aqueous dispersion according to any one of claims 1 to 8.

16. An adhesion preventing composition comprising the aqueous dispersion according to any one of claims 1 to 8.
